# EUROPEAN PATENT SPECIFICATION

(11) **EP 1 601 295 B1**
(45) Date of publication and mention of the grant of the patent: **16.12.2009**
(21) Application number: 04720091.0
(22) Date of filing: 12.03.2004
(51) Int. Cl.: A61B 17/16

(54) **AN ASSEMBLY FOR USE IN ORTHOPAEDIC SURGERY**
ANORDNUNG FÜR DIE ORTHOPÄDISCHE CHIRURGIE
ENSEMBLE UTILISABLE EN CHIRURGIE ORTHOPEDIQUE

(30) Priority: 13.03.2003 GB 0305777
(43) Date of publication of application: 07.12.2005
(73) Proprietor: Depuy International Limited, Beeston, Leeds LS11 8DT (GB)
(72) Inventor: GOODWIN, Peter, Lincoln LN6 5UH (GB); ORTON, Marcus, West Yorkshire LS23 6PB (GB)
(74) Representative: Belcher, Simon James
(86) International application number: PCT/GB2004/001110
(87) International publication number: WO 2004/080315

(56) References cited:
- EP-A- 0 782 840
- US-A- 4 023 572
- US-A- 4 906 147
- US-A- 5 018 266
- US-A1- 2002 099 380
- US-B1- 6 264 647

## Description

This invention relates to an assembly for use in orthopaedic surgery, which comprises a component having a bar extending across it, and a manipulator having a clasp for engaging the bar so as to fasten the component to the manipulator.

Hollow shell components have uses in orthopaedic surgery such as shaping bone tissue, for example to receive a component of an orthopaedic joint prosthesis, and as orthopaedic joint prosthesis components. An instrument having a hollow shell configuration has to be manipulated when used to shape a bone. For example, when the instrument is a cutting tool with cutting teeth on its external surface, it can be rotated about an axis of symmetry (for example when the external surface defines part of a sphere) to cause the bone tissue to be cut. A component of a joint prosthesis has to be manipulated to ensure that it is aligned properly with the prepared surface of the bone.

It is known to provide a hollow shell component with a bar which extends across it which can be engaged by a manipulator with an appropriate clasp. In the case of an instrument, the bar does not need to be detached from the shell, and can be bonded to the shell or formed integrally with it, for example by casting. In the case of a component of a joint prosthesis, the bar can be attached to the shell component by means of appropriate formations, for example which engage a lip on the component.

It is known to fasten a component to a manipulator by means of a clasp which comprises a hook and a keeper pin, in which the keeper pin can be retracted between an open position in which a bar on the component can be slid under the hook, and a closed position in which the keeper pin prevents the bar from being withdrawn from under the hook. The hook is provided on a plate at the end of a shaft, and the keeper pin slides through an aperture in that plate. The keeper pin is mounted on a collar which can slide along the shaft, and is resiliently biassed towards the plate. Connecting the component to the manipulator requires an operator to grip the collar and to slide it along the shaft, while at the same time moving the component so that the bar slides under the hook. This can be cumbersome.

Another similar instrument assembly is disclosed in US 2002 0099380.

The present invention provides an assembly for use in orthopaedic surgery, in which at least one of the contacting surfaces of the bar portion and the hook which contact one another when the bar portion is inserted between the hook and the keeper pin, or the surface of the bar portion which contacts the keeper pin, provides a ramp so that sliding the bar portion between the hook and the keeper pin causes one of the hook and the keeper pin to be displaced relative to the other, towards the open position.

Accordingly, in one aspect, the invention provides an instrument assembly for use in orthopaedic surgery, as defined in claim 1.

The assembly of the invention has the advantage that the bar on the component can be made to engage the clasp by inserting the bar between the hook and the keeper pin and sliding it under the hook. The provision of the ramp on one of the surfaces of the bar portion and the opposite surface of the clasp (whether the hook or the keeper pin) which contact one another can cause displacement of the hook or the keeper pin to as to open the clasp and to allow the bar to be received in the clasp. This avoids the need to grip a separate actuator (such as a sliding collar) to cause the said displacement.

Preferably, the hook is located on a plate so that the bar portion fits between the hook and the surface of the plate when the component is fastened to the manipulator, and in which the keeper pin extends through an aperture in the plate. The plate need not have a continuous surface and can have openings in it. It should provide a surface in at least those regions in which it will be engaged by the bar when the bar is located within the recess. For example, when the manipulator comprises two or more clasps which are arranged on it rotationally symmetrically, the plate can be in the form of a ring. The ring can have apertures formed in it, including for example apertures through which the keeper pins for the clasps extend.

Preferably, the bar which has a non-round cross-section. Preferably at least one surface of the bar is essentially planar, and the surface of the clamp which is engaged by the said planar surface of the bar is also planar. When the ramp surface is provided on the hook portion of the clasp, the bar can have two opposite surfaces which are substantially parallel; for example, the bar can have a rectangular cross-section.

The dimensions of the bar will depend on the size of the component and its intended use. The component might have a transverse dimension (which will generally correspond to the length of the bar) of, say, 20 to 50 mm. Preferably the width of the bar (over that portion of its length where the width is constant) can be at least about 2 mm, more preferably at least about 4 mm, for example at least about 5 mm. The width will generally be not more than about 10 mm, preferably not more than about 7 mm. The depth of the bar (over that portion of its length where the depth is constant) can be at least about 1 mm, more preferably at least about 1.5 mm, for example at least about 2 mm. The width will generally be not more than about 4 mm.

The ramp can be provided by the surface of the hook which contacts the bar portion when the bar portion is inserted between the hook and the keeper pin. For example, when the hook is located on a plate, the distance from the plate to the surface of the hook which faces towards the plate will be greatest at or towards the end of the hook which is first engaged by the bar portion. The thickness of the hook can vary along its length, for example so that the thickness of the hook increases along its length from around the end of the hook which is first engaged by the bar portion.

Preferably, the surface of that part of the assembly (especially one of the surfaces of the bar or a surface of the hook which faces the bar) which provides the ramp also provides an adjacent locked portion which engages a mating surface of another component when the component is fastened to the manipulator. For example, the hook can include a ramp portion which engages the bar portion while the bar portion is inserted between the hook and the keeper pin, and a locked portion which engages the bar portion which the component is fastened to the manipulator. The angle between the locked portion of the surface and the surface of the bar portion is smaller than the angle between the ramp portion and the surface of the bar portion Preferably, the locked portion of the surface and the surface of the bar portion are approximately parallel to one another.

The ramp can be provided by the surface of the bar portion which contacts the hook when the bar portion is inserted between the hook and the keeper pin. The surface of the bar portion can include a locked portion which contacts the hook when the component is fastened to the manipulator. The angle between the locked portion of the surface and the surface of the hook is smaller than the angle between the ramp portion and the surface of the hook. Preferably, the locked portion of the surface and the surface of the hook are approximately parallel to one another.

The ramp can be provided by the surface of the bar portion which contacts the keeper pin when the bar portion is inserted between the hook and the keeper pin. The surface of the bar portion can include a locked portion which contacts the keeper pin when the component is fastened to the manipulator. The angle between the locked portion of the surface and the surface of the keeper pin is smaller than the angle between the ramp portion and the surface of the keeper pin. Preferably, the locked portion of the surface and the surface of the keeper pin are approximately parallel to one another.

For example, the thickness of the bar portion can increase from the edge which engages the hook and the keeper pin when the bar portion is inserted between the hook and the keeper pin.

The manipulator can include a shaft on which the or each clasp is mounted. Preferably, the hook part of the clasp moves relative to the shaft during the said relative displacement between open and closed positions. For example, when the hook is provided on a plate, the plate can be arranged to slide relative to the shaft along the shaft axis.

An advantage of providing a hook which can be moved relative to a keeper pin is that the bar portion can be removed from within the hook easily using one hand, by applying twisting and pulling forces to the component relative to the manipulator.

Preferably, the manipulator includes at least two clasps which are arranged rotationally symmetrically around a central point, and the component includes corresponding bar portions, so that the bar portions can be positioned in corresponding clasps by relative rotation between the component and the manipulator around the said central point. For example, the manipulator can include two clamps which are arranged at diametrically opposite sides of a plate. The hooks should face in the same direction rotationally so that the bar portions are received in the respective hooks in a single rotational movement. There can be more than two clasps, for example three or four clasps. Preferably, these are spaced apart equally around a central point.

The external surface of the component can be symmetrical about an axis of rotation. For example, the external surface can define a part of a sphere. This might be the case when the assembly is for use in connection with a joint prosthesis in which one component articulates against the other component in the manner of a ball which is received in a cup. It is envisaged that the component in the assembly of the invention can be a component of a joint prosthesis, for example the cup component of a joint prosthesis, especially the cup component of a hip joint prosthesis or a shoulder joint prosthesis. While the configuration of the external surface can preferably be symmetrical about an axis of rotation, the component need not be symmetrical in this way. For example, the component can have one or more cut-out portions. The wall thickness of the component can vary from one region to another. The component can have features on its outer surface according to its intended purpose: for example, the component might be a cutting tool, in which case it can have cutting teeth on its outer surface, and the arrangement of the teeth on the surface need not necessarily be symmetrical about the axis of symmetry.

When the component is a cutting tool, the use of a rotationally symmetrical component has the advantage that the tool can be rotated about the axis to cut the patient's bone tissue. When the external surface of the component defines a part of a sphere, the tool can be rotated about its axis to cut the patient's bone tissue, while at the same time the orientation of the axis relative to the bone is changed. This can be important to achieve satisfactory cutting of the patient's bone in preparation for implantation of a joint prosthesis component. Preferably, the bar to which the clasp fastens intersects the axis of symmetry of the component.

Preferably, the assembly of the invention includes a drive mechanism for imparting rotational drive to the component. This can be particularly useful when the component is an instrument such as a cutting tool. The manipulator can comprise a hollow sleeve, which has a rod (which might itself be hollow) within it. The rod can be driven rotationally relative to the sleeve. The component can be mounted on the rod. Rotational drive can be imparted to the component can be imparted manually, for example using a handle. Alternatively, a powered drive unit can be used. The hollow sleeve can be used to hold the manipulator, either directly, or using a handle which is attached to the sleeve.

Preferably, the component is hollow and has an open face. Preferably, the portion of the bar which is engaged by the clasp is located within the component so that, when the component is fastened to the manipulator, the clasp is located at least partially within the component.

The bar which extends across the component can be straight (when viewed along a line perpendicular to the axis of the component and to the bar), in which case, it will be fastened to the internal wall of the component at its ends, at the same depth as point at which the clasp engages the bar. The bar can be cranked, so that the depth within the component of the ends of the bar where they engage the component need not be the same as the depth of the point at which the clasp engages the bar. For example, the ends of the bar can be located closer to the open side of the component than the point at which the clasp engages the bar, for example with the ends of the bar fastened to the component at the open side. The ends of the bar can be located further from the open side of the component than the point at which the clasp engages the bar; for example the ends of the bar can be fastened to the internal surface of the component close to the pole of the component. The bar can also be mounted on a fixture which is fastened to the internal wall of the component at or close to the pole: for example, the fixture can comprise a length of a tube, and the bar extends across the tube.

The bar is preferably straight when viewed along the axis of the component. The bar can include more than one limb or there can be more than one bar. For example, there can be two bars which are fastened together at about the component axis, so that there are four bar portions extending radially from the axis. The bar portions can then be perpendicular to one another at the point at which they are fastened together. Other arrangements are envisaged, for example in which the bar is provided by three bar portions which are joined together so that the angle between any two of the bar portions is about 120°. The bar portions can be joined together at or close to the axis of the component. They might however not extend to the axis of the component and be joined together by means of a ring which encircles the axis.

Embodiments of the present invention will now be described by way of example with reference to the accompanying drawings in which:
Figure 1 is an isometric view of a shell component of an assembly according to the invention, within hidden detail shown in dotted outline.
Figure 2 is an exploded isometric view of the head of a manipulator and a component such as that shown in Figure 1.
Figure 3 is an exploded isometric view of the head of the manipulator shown in Figure 2 and having a component connected to it.
Figures 4 to 6 are sequential isometric side views of an assembly according to the invention with the shell component in different angular orientations relative to the manipulator as a connection is formed between the component and the manipulator.
Figure 7 is a view from above of the clasp mechanism of the manipulator and the bar of the component when the component is connected to the manipulator.

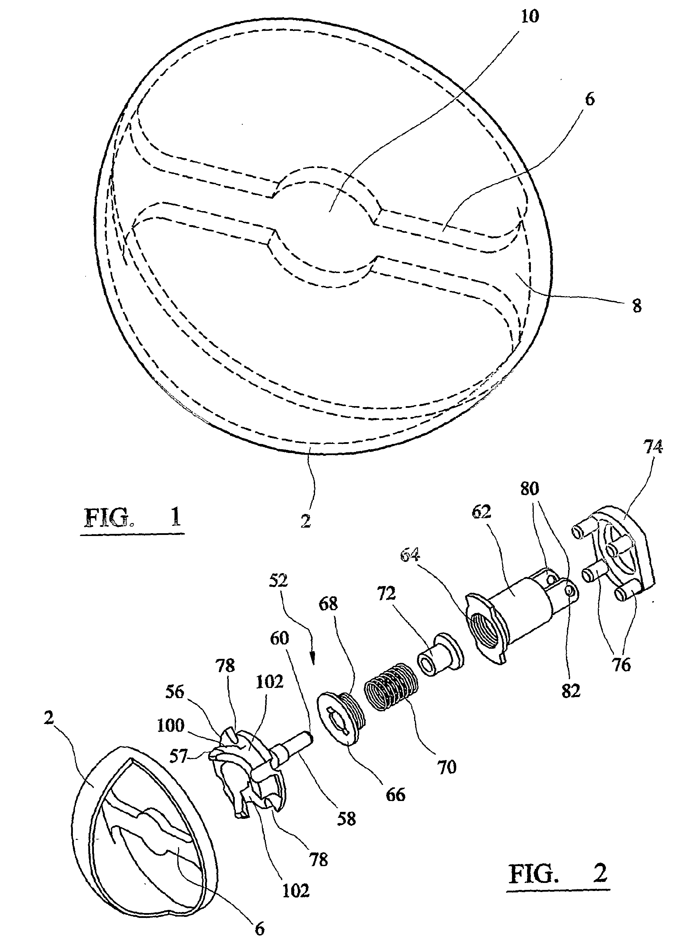

Referring to the drawings, Figure 1 shows a shell component of a kind which can be used in the orthopaedic surgery, for example in the implantation of the acetabular cup component of a hip joint prosthesis or the implantation of a component of a shoulder joint prosthesis. The component can be an implant. It can be an instrument which is used to prepare the patient's tissue to receive the implant, for example a reamer.

The component shown in Figure 1 comprises a hollow shell 2 formed from a suitable metallic material (for example a stainless steel). When the shell is a reamer, it has raised cutting teeth arranged on its surface. The external surface of the shell defines a part of a sphere and the open side of the shell is at or slightly above about the equatorial plane of that sphere, so that the shell is symmetrical about the polar axis of the shell. Each of the cutting teeth is directed so as to cut the surface of a bone when rotated about the axis of symmetry. The shell is provided by a thin sheet of the metallic material, and can be manufactured by forming a sheet, or by other techniques such as casting and appropriate subsequent finishing.

The reamer component shown in Figure 1 has a bar 6 within it. The bar is fastened at its ends to the internal surface of the shell. It can be fastened by welding; it can be formed with other parts of the shell be machining, for example with a collar part from a sheet. The bar is straight, when viewed along the axis of the sphere, and also when viewed from one side.

The bar is rectangular in cross-section over most of its length, apart from the end parts 8 where it is fastened to the shell part and from a central portion 10 where the bar is enlarged.

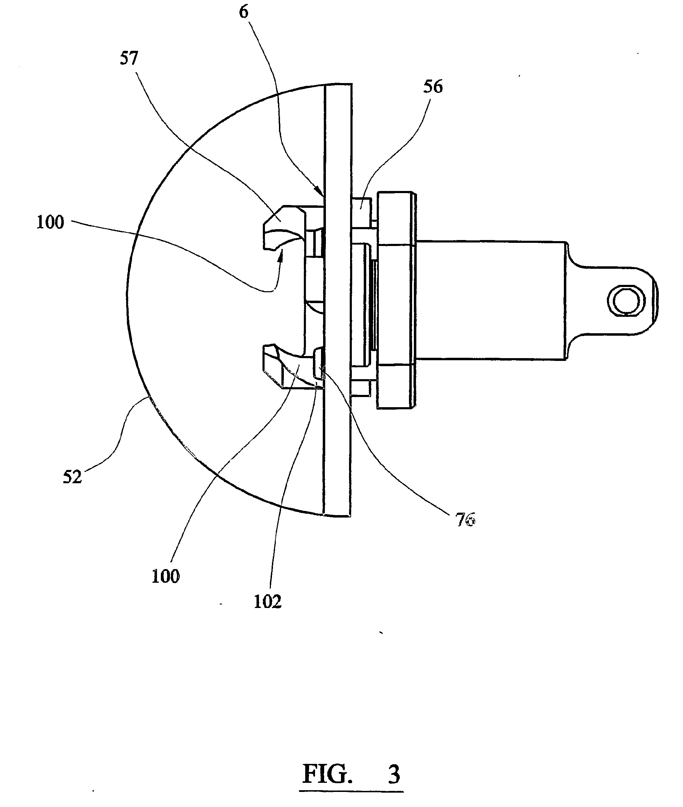

Figures 2 and 3 show an assembly according to the invention which comprises a shell component 2 and a manipulator 52 (of which the head only is shown in Figures 2 and 3). The head of the manipulator comprises a plate 56 which carries two hooks 57 arranged diametrically opposite to one another on the plate. The plate also carries a downwardly extending spigot 58 which is threaded at its distal end 60. The spigot can be received in a bearing sleeve 62 which is threaded at its upper end 64. The bearing sleeve can be closed by means of a closure cap 66 which carries an external thread 68 which engages a thread on the internal surface of the bearing sleeve at its upper end. A spring 70 is provided on the spigot, which is held in place on the spigot by means of a bearing cap 72. The bearing cap is a sliding fit within the sleeve, allowing the plate 56 to be displaced upwardly relative to the bearing sleeve, causing compression of the spring 70 between the bearing cap and the closure cap.

The bearing sleeve 62 can fit in a central opening in a collar 74. The collar has four pins 76 extending upwardly from it. The plate 56 has openings 78 in it through which the pins can extend. Displacement of the plate 56 relative to the bearing sleeve causes the pins to move in the openings 78 in the plate. The extension of the pins through the openings in the plate is at a maximum when the plate is in the locked position, in which the underside of the plate 56 is in contact with the closure cap 66. The spacing between the free ends of the pins 76 and the free end of the respective hooks 57 is slightly greater than the depth of the bar 6 on the component so that the bar can be slid into the space between the end of a hook and the end of the pin which is adjacent to the end thereof.

The bearing sleeve has a pair of attachment plates 80 at its lower end, each with an opening 82 extending through it. The openings can receive a fastener such as a machine screw to fasten the bearing sleeve to an elongate member such as a rod (which might be hollow in the form of a tube or sleeve). The rod can fit within a tubular shaft so that it can rotate relative to the shaft, in use to impart rotational movement to the bearing sleeve and the plate relative to the shaft. The assembly can be gripped directly on the shaft or indirectly using a handle which is connected to the shaft.

Each of the hooks 57 has a ramp portion 100 in which the distance between the plate 56 and the surface of the ramp portion of the hook which faces towards the plate decreases from the free end of the hook. The angle between the ramp portion and the surface of the plate is not more than about 40°, preferably not more than about 30°, more preferably not more than about 20°, for example about 15°.

Each of the hooks 57 includes a locked portion 102 at or towards the root 103 of the hook. The distance between the plate 56 and the surface of the locked portion of the hook which faces towards the plate is substantially constant across the locked portion, and is such that the bar is a snug fit between the plate and the locked portion of the hook.

Figure 3 shows the shell component 2 connected to the manipulator, with the bar 6 located between the hooks 57 and the surface of the plate 56, where they are held by means of the pins 76.

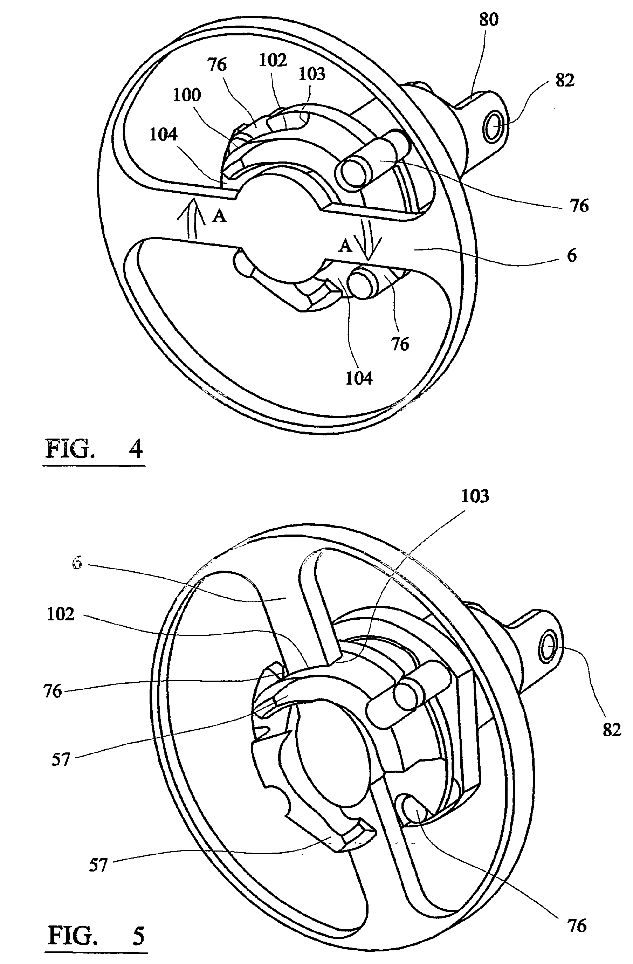

In order to fasten the shell component 2 to the head of the manipulator, the component is offered to the manipulator with the bar 6 facing the plate 56. The bar is slid into the space 104 between the free ends of the hooks 57 and the adjacent pins76 by rotating the component relative to the manipulator. This is shown in Figure 4, with the direction of rotation of the shell component indicated by the arrow A.

Continued rotation of the shell relative to the manipulator causes the bar to contact the ramp portion 100 of the hook. As the shell is rotated further, action of the surface 106 of the bar which faces away from the plate against the ramp portion of the hook causes the plate 56 (on which the hooks are fixed) to be displaced relative to the closure cap 66, with compression of the spring 70 between the closure cap and the bearing cap 72. This displacement continues until the locked portions 102 of the hook surfaces engages the surface 106 of the bar which faces away from the plate. This is shown in Figure 5.

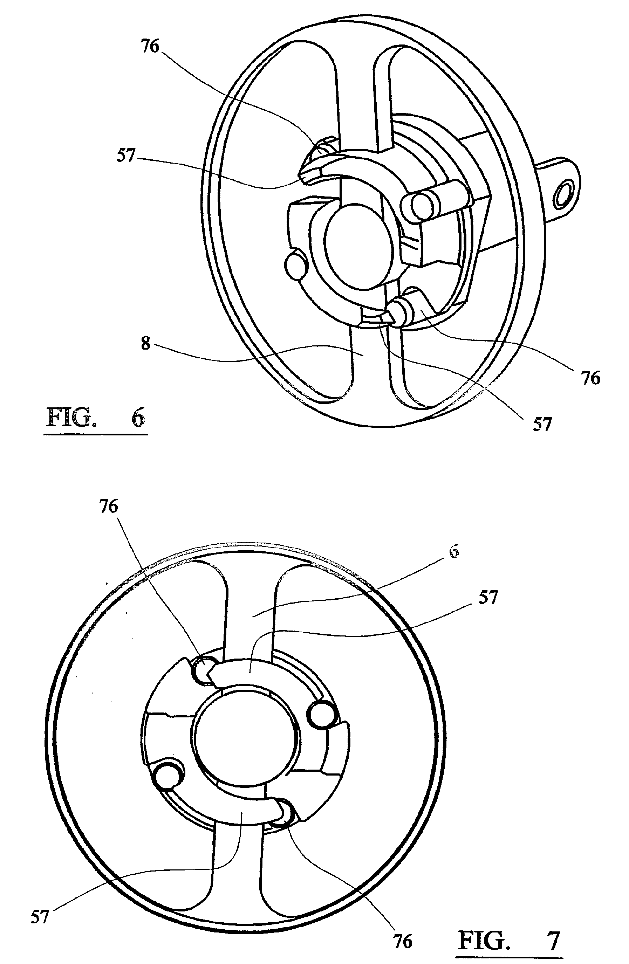

The distance between the root 103 of each of the hooks and the corresponding pin is slightly greater than the width of the bar 6 on the shell component. Once the bar is in contact with the roots of the hooks, the bar and the plate are then able to move relative to the closure cap 66 and the pins 76 until the plate is in contact with the closure cap and the spring 70 is relaxed. The bar is then a snug fit between the roots of the hooks and the pins against rotational movement relative to the plate. This is shown in Figures 6 and 7. When locked in this way against relative rotational movement, rotational movement can be imparted to the shell component by means of a drive unit which is connected to the bearing sleeve by means of a fastener inserted in the openings 82 in the attachment plages 80.

The component can be detached from the manipulator by lifting it and the plate relative to the closure cap, against the force exerted by the spring 70 as it is compressed. When the plate has been lifted through a distance which is no less than the thickness of the bar 6, the component can be twisted relative to the plate, over the pins.

The assembly of the present invention can be used to manipulate other components. For example, it can be used to manipulate instruments other than reamers. It can be used to manipulate implant components, for example the acetabular cup component of a hip joint prosthesis. Generally, the prosthesis will require a suitable mounting for the fastening formation.

## Claims

1. An instrument assembly for use in orthopaedic surgery, which comprises a component (2) which is suitable to be positioned within a body cavity to engage a bone and which has at least one bar portion (6) extending across it, and a manipulator (52) having at least one clasp for engaging the bar portion (6) so as to fasten the component to the manipulator (52), the clasp comprising a hook (57) and a keeper pin (76), in which:
a. the hook (57) is suitable to be displaced relative to the keeper pin (76) between an open position in which the keeper pin (76) is retracted relative to the hook (57) so that the hook (57) is open at one side to allow the bar portion (6) to be slid between the hook (57) and the keeper pin 76, and a closed position in which the keeper pin (76) closes the hook (57) sufficiently to prevent the bar portion (6) from being removed from under the hook,
b. at least one of the contacting surfaces of the bar portion (6) and the hook (57) which are suitable to contact one another when the bar portion (6) is inserted between the hook (57) and the keeper pin (76), or the surface of the bar portion (6) which contacts the keeper pin (76), providing a ramp (100), so that sliding the bar portion (6) between the hook (57) and the keeper pin (76) causes the hook to be displaced relative to the keeper pin (76), towards the open position, and
c. the hook (57) suitable to be is biased towards the closed position.

2. An instrument assembly as claimed in claim 1, in which the hook (57) is located on a plate (56) so that the bar portion (6) fits between the hook (57) and the surface of the plate (56) when the component (2) is fastened to the manipulator, and in which the keeper pin (76) extends through an aperture in the plate.

3. An instrument assembly as claimed in claim 1, in which the manipulator (52) includes a shaft on which the clasp is mounted, in which the hook (57) part of the clasp is suitable to move relative to the shaft during the said relative displacement between open and closed positions.

4. An instrument assembly as claimed in claim 1, in which the ramp (100) is provided by the surface of the hook (57) which is suitable to contact the bar portion (6) when the bar portion (6) is inserted between the hook (57) and the keeper pin (76).

5. An instrument assembly as claimed in claim 4, in which the hook (57) includes a ramp portion (100) which is suitable to engage the bar portion (6) while the bar portion (6) is inserted between the hook (57) and the keeper pin (76), and a locked portion (102) which is suitable to engage the bar portion (6) which the component (2) is fastened to the manipulator (52).

6. An instrument assembly as claimed in claim 1, in which the manipulator (52) includes at least two clasps which are arranged rotationally symmetrically around a central point, and the component includes corresponding bar portions, so that the bar portions can be positioned in corresponding clasps by relative rotation between the component and the manipulator around the said central point.

## Patentansprüche

1. Instrumentenbaugruppe zur Verwendung bei der orthopädischen Chirurgie, die eine Komponente (2), welche zur Anordnung innerhalb eines Körperhohlraums, um einen Knochen zu greifen, geeignet ist und welche wenigstens einen Stangenbereich (6) hat, der sich über sie erstreckt, und einen Manipulator (52) mit wenigstens eine Schließelement zum Eingriff mit dem Stangenabschnitt (6), um so die Komponente an dem Manipulator (52) zu befestigen, hat, wobei das Schließelement einen Haken (57) und einen Haltestift (76) aufweist, wobei:
a. der Haken (57) zur Verlagerung in Bezug auf den Haltestift (76) zwischen einer Offenstellung, in der der Haltestift (76) in Bezug auf den Haken (57) zurückgezogen ist, so dass der Haken (57) an einer Seite offen ist, um zu ermöglichen, dass der Stangenbereich (6) zwischen dem Haken (57) und dem Haltestift (76) zu verschieben ist, und einer Schließstellung, in der der Haltestift (76) den Haken (57) ausreichend verschiebt, um zu verhindern, dass der Stangenbereich (16) unter dem Haken heraus entfernt wird, geeignet ist,
b. wenigstens eine der Kontaktflächen des Stangenbereiches (6) und des Hakens (57), die dazu geeignet sind, einander zu berühren, wenn der Stangenbereich (6) zwischen den Haken (57) und den Haltestift (76) geführt ist, oder die Fläche des Stangenbereiches (6), die den Haltestift (76) berührt, eine Rampe (100) bildet, so dass das Verschieben des Stangenbereiches (6) zwischen dem Haken (57) und dem Haltestift (76) bewirkt, dass der Haken in Bezug auf den Haltestift (76) auf die offene Position zu verlagert wird, und
c. der Haken (57) dazu geeignet ist, auf die Schließstellung zu vorbelastet zu werden.

2. Instrumentenbaugruppe nach Anspruch 1, bei der sich der Haken (57) auf einer Platte (56) befindet, so dass der Stangenbereich (6) zwischen den Haken (57) und der Oberfläche der Platte (56) eingepasst ist, wenn die Komponente (2) an dem Manipulator befestigt ist, und bei der sich der Haltestift (76) durch eine Öffnung in der Platte erstreckt.

3. Instrumentenbaugruppe nach Anspruch 1, bei der der Manipulator (52) einen Schaft umfasst, auf dem das Schließelement angeordnet ist, wobei der Haken (57)-Teil der Klammer dazu geeignet ist, sich in Bezug auf den Schaft während der relativen Verlagerung zwischen der Offen- und der Schließstellung zu bewegen.

4. Instrumentenbaugruppe nach Anspruch 1, bei der die Rampe (100) durch die Oberfläche des Hakens (57) gebildet wird, die dazu geeignet ist, den Stangenbereich (6) zu berühren, wenn der Stangenbereich (6) zwischen den Haken (57) und den Haltestift (76) geführt ist.

5. Instrumentenbaugruppe nach Anspruch 4, bei der der Haken (57) einen Rampenbereich (10), der dazu geeignet ist, den Stangenbereich (6) zu greifen, während der Stangenbereich (6) zwischen den Haken (57) und den Haltestift (76) eingeführt wird, und einen verriegelten Bereich (102), der dazu geeignet ist, den Stangenbereich (6) zu greifen, wenn die Komponente (2) an dem Manipulator (52) befestigt ist, umfasst.

6. Instrumentenbaugruppe nach Anspruch 1, bei der der Manipulator (52) wenigstens zwei Schließelemente hat, die drehsymmetrisch um einen Mittelpunkt angeordnet sind, und die Komponente entsprechende Stangenbereiche umfasst, so dass die Stangenbereiche in entsprechenden Schließelementen durch die relative Drehung zwischen der Komponente und dem Manipulator um den Mittelpunkt positioniert werden können.

## Revendications

1. Ensemble d'instrument destiné à être utilisé en chirurgie orthopédique, qui comprend un composant (2) qui est approprié pour être placé à l'intérieur d'une cavité du corps de manière à venir en prise avec un os, et qui présente au moins une partie de barre (6) qui s'étend à travers celui-ci, et un dispositif de manipulation (52) qui présente au moins une attache destinée à venir en prise avec la partie de barre (6) de manière à attacher le composant sur le dispositif de manipulation (52), l'attache comprenant un crochet (57) et une broche de retenue (76), dans lequel :
a. le crochet (57) est approprié pour être déplacé par rapport à la broche de retenue (76) entre une position ouverte dans laquelle la broche de retenue (76) est rétractée par rapport au crochet (57) de telle sorte que le crochet (57) soit ouvert au niveau d'un côté de manière à permettre à la partie de barre (6) de coulisser entre le crochet (57) et la broche de retenue (76), et une position fermée dans laquelle la broche de retenue (76) ferme le crochet (57) suffisamment de manière à empêcher la partie de barre (6) d'être retirée de dessous le crochet,
b. l'une au moins des surfaces de contact de la partie de barre (6) et du crochet (57) qui sont appropriées pour entrer en contact l'une avec l'autre lorsque la partie de barre (6) est insérée entre le crochet (57) et la broche de retenue (76), ou la surface de la partie de barre (6) qui entre en contact avec la broche de retenue (76), fournissant une rampe (100), de telle sorte que le coulissement de la partie de barre (6) entre le crochet (57) et la broche de retenue (76) provoque un déplacement du crochet par rapport à la broche de retenue (76), vers la position ouverte, et
c. le crochet (57) est approprié pour être sollicité vers la position fermée.

2. Ensemble d'instrument selon la revendication 1, dans lequel le crochet (57) se situe sur une plaque (56) de telle sorte que la partie de barre (6) s'ajuste entre le crochet (57) et la surface de la plaque (56) lorsque le composant (2) est fixé sur le dispositif de manipulation, et dans lequel la broche de retenue (76) s'étend à travers une ouverture située dans la plaque.

3. Ensemble d'instrument selon la revendication 1, dans lequel le dispositif de manipulation (52) comprend une tige sur laquelle est montée l'attache, dans lequel la partie de crochet (57) de l'attache est appropriée pour se déplacer par rapport à la tige au cours dudit déplacement relatif entre les positions ouverte et fermée.

4. Ensemble d'instrument selon la revendication 1, dans lequel la rampe (100) est fournie par la surface du crochet (57) qui est appropriée de manière à entrer en contact avec la partie de barre (6) lorsque la partie de barre (6) est insérée entre le crochet (57) et la broche de retenue (76).

5. Ensemble d'instrument selon la revendication 4, dans lequel le crochet (57) comprend une partie de rampe (100) qui est appropriée de manière à venir en prise avec la partie de barre (6) tandis que la partie de barre (6) est insérée entre le crochet (57) et la broche de retenue (76), et une partie verrouillée (102) qui est appropriée de manière à venir en prise avec la partie de barre (6) à laquelle le composant (2) est fixé sur le dispositif de manipulation (52).

6. Ensemble d'instrument selon la revendication 1, dans lequel le dispositif de manipulation (52) comprend au moins deux attaches qui sont disposées en rotation de manière symétrique autour d'un point central, et le composant comprend des parties de barre correspondantes, de telle sorte que les parties de barre puissent être placées dans des attaches correspondantes par une rotation relative entre le composant et le dispositif de manipulation autour dudit point central.
